(19) Europäisches Patentamt
European Patent Office
Office européen des brevets

(11) **EP 3 972 663 B1**

(12) **EUROPÄISCHE PATENTSCHRIFT**

(45) Veröffentlichungstag und Bekanntmachung des Hinweises auf die Patenterteilung:
**06.03.2024   Patentblatt 2024/10**

(21) Anmeldenummer: **20727623.9**

(22) Anmeldetag: **20.05.2020**

(51) Internationale Patentklassifikation (IPC):
**A61M 1/16** (2006.01)        **A61M 1/36** (2006.01)
**G01L 27/00** (2006.01)

(52) Gemeinsame Patentklassifikation (CPC):
**A61M 1/3639; A61M 1/1605; G01L 27/002**

(86) Internationale Anmeldenummer:
**PCT/EP2020/064175**

(87) Internationale Veröffentlichungsnummer:
**WO 2020/234407 (26.11.2020 Gazette 2020/48)**

(54) **DRUCKMESSUNG IM EXTRAKORPORALEN BLUTKREISLAUF**

PRESSURE MEASUREMENT IN THE EXTRACORPOREAL BLOOD CIRCUIT

MESURE DE PRESSION DANS UN CIRCUIT SANGUIN EXTRA-CORPOREL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.05.2019   DE 102019113561**

(43) Veröffentlichungstag der Anmeldung:
**30.03.2022   Patentblatt 2022/13**

(73) Patentinhaber: **B. Braun Avitum AG**
**34212 Melsungen (DE)**

(72) Erfinder:
• **AHMED, Fakher**
**64319 Pfungstadt (DE)**
• **KRAUSE, Silvie**
**34212 Melsungen-Adelshausen (DE)**
• **WOLFF, Dr. Henrik**
**34212 Melsungen-Adelshausen (DE)**
• **WÜRSCHMIDT, Tobias**
**34346 Hann. Münden (DE)**

(74) Vertreter: **Winter, Brandl - Partnerschaft mbB**
**Alois-Steinecker-Straße 22**
**85354 Freising (DE)**

(56) Entgegenhaltungen:
EP-A1- 2 725 335        DE-A1- 19 747 254
DE-A1-102015 109 450    DE-C2- 19 747 254
US-B1- 6 503 062

**Beschreibung**

Technisches Gebiet

Die vorliegende Offenbarung betrifft ein Verfahren sowie eine Vorrichtung zur Kalibrierung von einer Druckmessung bzw. von Kraftsensor-Messwerten zur Bestimmung eines Schlauchinnendrucks im extrakorporalen Kreislauf zur Korrektur des durch die Druckmessung bestimmten Messwertes mit einem Korrektursignal bei Verwendung von direkt an einem befüllten Schlauch anliegenden Kraftsensoren.

Stand der Technik

Allgemein ist es möglich, einen Schlauchinnendruck mithilfe einer Druckmessleitung zu messen. Dabei ist der (erste) Schlauch, dessen Innendruck gemessen werden soll, mit einer Druckmessleitung (zweiter Schlauch) verbunden, die wiederum den zu messenden Druck an einen (piezoelektrischen) Drucksensor/Druckaufnehmer leitet. Die Druckmessleitung ist hierfür über ein T-Stück mit dem (ersten) Schlauch verbunden. Am oberen (freien) Ende der Druckmessleitung ist der Drucksensor vorzugsweise mit Luer-Lock-Anschluss angeordnet. Zwischen einer in der Druckmessleitung stehenden Flüssigkeitssäule und dem Druckaufnehmer befindet sich ein Luftpolster, welches sich bei Druckänderung im (ersten) Schlauch verändert (ausdehnt oder kleiner wird), was wiederum zu einer entsprechenden Auslenkung des Druckaufnehmers führt.

Ein derartiges Druckmessverfahren bzw. Messsystem bringt unter anderem die Nachteile mit sich, dass im Falle, dass durch den (ersten) Schlauch beispielsweise Blut im Zusammenhang mit einem extrakorporalen Kreislauf oder eine andere Luftoxidierende Flüssigkeit strömt, ein Flüssigkeit-Luft Kontakt innerhalb der Druckmessleitung entsteht, die Herstell- und Montagekosten des Schlauchs aufgrund des T-Stücks ansteigen und die Reinigung der Druckmessleitung durch diesen Messaufbau erschwert wird. Darüber hinaus besteht die Gefahr eines direkten Kontaktes des Drucksensors/Druckaufnehmers mit der Flüssigkeit.

Um beispielsweise einen Blut-Luft-Kontakt zu vermeiden, der vor allem bei Anwendungen wie bei einer Dialyse nachteilig ist, werden sogenannte "Pressure Pods" verwendet. Dabei erfolgt die Druckübertragung nicht direkt von beispielsweise Blut auf das Luftpolster, sondern Blut und Luft sind über eine flexible Membran voneinander getrennt. Durch eine Druckänderung innerhalb des (ersten) Schlauchs wird die Membran ausgelenkt und diese Krafteinwirkung wird über ein an die Membran sich anschließendes Luftpolster auf einen Drucksensor übertragen, welcher den Schlauchinnendruck misst. D.h., dass auch bei dieser bekannten Konstruktion ein Luftpolster als Druckübertragungsmedium zwischen Membran und Drucksensor vorgesehen ist, wenngleich ein direkter Kontakt zwischen dem Luftpolster und dem in dem (ersten) Schlauch strömendem Fluid vermieden wird. Dieser Messaufbau hat zudem den Nachteil, dass er hohe Herstellungskosten mit sich bringt.

Daher ist beispielsweise in der EP 1 357 372 A1 eine Klemmvorrichtung vorgesehen, in welcher ein (ersten) Schlauch verklemmt wird, dessen Innendruck gemessen werden soll. Die Schlauchinnendruckmessung erfolgt nichtinvasiv, d.h. mithilfe einer Kraftmessung über die Schlauchaußenwand und nicht bspw. über eine T-Verzweigung zur Verbindung zwischen dem Gefäßinneren und einer Messsensorik. Die Ausdehnung des Schlauchs aufgrund einer Schlauchinnendruckänderung wird über ein Kraftübertragungsmittel auf einen Kraftsensor übertragen, der ein Kraftsignal ausgibt. Die Änderung des Kraftsignals wird mithilfe eines Proportionalitätsfaktors proportional in eine Druckänderung umgerechnet. Bei einer Druckänderung wird somit nur die Schlauchverformung an einem durch einen Stützkörper der Klemmvorrichtung längs sich erstreckenden Spalt zur Erzeugung des Kraftsignals ausgewertet.

Allerdings ist bei der Schlauchinnendruckmessung über die Klemmvorrichtung das viskoelastische Verhalten des Schlauchs zu beachten. D.h., beim Einklemmen des Schlauchs entsteht eine Rückstellkraft in Form eines Driftsignals, welches das zu messende Kraftsignal bzw. Drucksignal überlagert. Bei einer längeren Messzeit wirkt sich die Rückstellkraft so aus, dass der Innendruck im (ersten) Schlauch auch bei konstanten Bedingungen scheinbar abfällt. Zur Lösung dieses Problems wurde bisher angenommen, dass das Drucksignal durch Referenzieren mit Umgebungsluft korrigiert werden kann. Das heißt, dass das viskoelastische Verhalten vor der aktiven Verwendung des Schlauchs durch das Befüllen des Schlauchs mit Luft untersucht wird. Das dabei erhaltene Rückstellsignal wird dann bei einer aktiven Verwendung vom angezeigten Druckverlauf (im mit einer Flüssigkeit, z.B. Blut, befüllten Schlauch) subtrahiert. Allerdings verhält sich ein mit Medium/Flüssigkeit befüllter Schlauch anders als im mit Luft befüllten Zustand. Daher gibt ein mit Umgebungsluft referenziertes Drucksignal keinen Hinweis auf das Drucksignal eines befüllten Schlauchs.

Um ein derart verfälschtes Drucksignal korrigieren zu können, sieht das Dokument DE 197 47 254 C2 ein Verfahren zur Korrektur eines Drucksignals vor, das über eine Klemmvorrichtung gemäß vorstehender Beschreibung gemessen wird. Der Verlauf der Rückstellkraft wird hierfür in Form einer Relaxationsfunktion wiedergegeben, welche schlauchabhängig ist und im Vorhinein bekannt ist (ermittelt wird). Die Parameter dieser Funktion werden aus dem gemessenen Kraftsignal ermittelt. Mithilfe dieser Relaxationsfunktion kann das Kraftsignal korrigiert und das Drucksignal über einen linearen Zusammenhang mit dem Kraftsignal ermittelt werden.

Kurzbeschreibung der Erfindung

Vor diesem Hintergrund ist es die Aufgabe der vorliegenden Erfindung, die Korrektur des Kraftsignals weiter zu verbessern und das durch die mechanischen Eigenschaften des (ersten) Schlauchs verursachte Driftsignal vor und während einer aktiven Verwendung des (ersten) Schlauchs (eine aktive Verwendung des Schlauchs bedeutet beispielsweise ein an einen Patienten angeschlossener Schlauch, bzw. ein Schlauch während einer Therapie) mithilfe eines Referenzsignals zu korrigieren. Weiterhin sollen relative Druckänderungen sowie auch der absolute Schlauchinnendruck bevorzugt mit einer Druckgenauigkeit von ± 10 mmHg bestimmt werden.

Diese Aufgabe wird durch ein Verfahren nach Anspruch 1 und eine Vorrichtung nach Anspruch 10 gelöst. Vorteilhafte Ausgestaltungen der Erfindung sind Gegenstand der Unteransprüche.

Die Erfindung sieht daher ein Verfahren zur Kalibrierung eines ersten Kraftsensors/eines ersten Drucksensors vor, der innerhalb eines mit Flüssigkeit befüllten (ersten) Schlauchs, vorzugsweise eines Dialysatorschlauchs (Blutschlauchs), einen ersten Druck, insbesondere einen arteriellen Druck, beispielsweise in einem extrakorporalen (Blut-)Kreislauf, in Form eines Kraftsignals misst. Der Drucksensor liegt direkt am (ersten) Schlauch an und ist in eine erste Klemmvorrichtung integriert/eingesetzt, um ein vom (ersten) Schlauch hervorgerufenes Driftsignal durch eine schlauchparameterunabhängige Korrekturfunktion mithilfe eines Druckreferenzsignals zu korrigieren, das durch einen ersten (separaten) Druckreferenzsensor aufgenommen wird. Erfindungsgemäß werden folgende Schritte durchgeführt:

a) Regressionsanalyse und Vorhersage zumindest einer schlauchparameterunabhängigen Korrekturfunktion zur Findung eines Korrektursignals zur Korrektur des Driftsignals mithilfe eines/des entsprechenden Druckreferenzsignals, das durch den ersten Druckreferenzsensor bei konstantem Schlauchinnendruck und konstanter Schlauchinnentemperatur gemessen wird;

b) erstes Kalibrieren des mit dem ersten Kraftsensor gemessenen und mithilfe des Korrektursignals korrigierten Kraftsignals mit dem Druckreferenzsignal, das durch den ersten Druckreferenzsensor gemessen wird, vor einer aktiven Verwendung des Schlauchs; und

c) zweites Kalibrieren des mit dem ersten Kraftsensor gemessenen und mithilfe der (zuvor vorhergesagten) Korrekturfunktion korrigierten Kraftsignals mit dem Druckreferenzsignal, das durch einen zweiten (separaten) Druckreferenzsensor gemessen wird, während einer aktiven Verwendung des Schlauchs.

In anderen Worten ist ein Verfahren zur Onlinekorrektur eines Kraft-Drucksignals vorgesehen, welches erfindungsgemäß auf einen befüllten (ersten) Schlauch vor und während einer aktiven Verwendung des (ersten) Schlauchs angewendet wird. D.h. es wird zunächst eine erste Kalibrierung eines von einem ersten Kraftsensor erzeugten (Test-/Simulations-)Kraftsignals vor einer aktiven Verwendung des (ersten) Schlauchs mittels eines Druckreferenzsignals durchgeführt, welches mittels eines Druckreferenzsensors sowie einer schlauchparameterunabhängigen Korrekturfunktion ermittelt wurde. Dann wird eine zweite Kalibrierung eines anhand der Korrekturfunktion bereits korrigierten (zweiten) Kraftsignals während einer aktiven Verwendung des Schlauchs durchgeführt und zwar auf Basis eines Druckreferenzsignals, das bevorzugt von einem zweiten Druckreferenzsensor erzeugt wird/wurde.

Im Konkreten werden hierfür eine mathematische Korrekturfunktion und eine ZweiPunkt-Kalibrierung so angewendet, dass eine absolute Druckmessung möglich ist. Die Druckmessung erfolgt über einen/den Kraftsensor, der in eine/die Klemmvorrichtung integriert ist. Die mathematische Korrekturfunktion wird bei konstantem Schlauchinnendruck und kontanter Schlauchinnentemperatur, vorzugsweise innerhalb weniger Minuten, nach dem Einlegen des (ersten) Schlauchs in die Klemmvorrichtung aus dem gemessenen (Text-)Kraft-Drucksignal und einem Referenzdruck ermittelt, der über einen/den (separaten) Druckreferenzsensor (welcher einen zum Kraftsensor unterschiedlichen Aufbau und/oder unterschiedlichen Einbau aufweist) ermittelt wird und liefert ein Driftsignal. Der Druckreferenzsensor weist bevorzugt eine höhere Messgenauigkeit als der entsprechende Druck-/Kraftsensor auf. Es wird dann eine/die erste Kalibrierung durchgeführt, bei der während der Aufnahme des Druck-/Kraftsignals zur Bestimmung der Korrekturfunktion der Schlauchinnendruck und die Schlauchinnentemperatur konstant sind. Bei einer darauffolgenden Rekalibrierung (zweite Kalibrierung) wird der (erste) Schlauch mit einem konstanten, bekannten Druck beaufschlagt und aus den in diesem Zeitraum gemessenen Druckdaten die Korrekturfunktion erneut bestimmt. Eine derartige Referenzmethode umfasst somit einen Kalibriervorgang sowoh vor einer aktiven Verwendung des Schlauchs also auch während einer aktiven Verwendung des Schlauchs, d.h. die Referenzmethode wird vor und während einer (Dialyse-)Therapie am Patienten, durchgeführt.

Eine derartiges Verfahren ermöglicht die Druckmessung über eine Klemmvorrichtung direkt am befüllten (ersten) Schlauch als Teil eines Schlauchsystems. Bei dieser Art der Druckmessung entfallen im Bereich der Klemmvorrichtung die im Stand der Technik verwendeten Luer-Lock-Anschlüsse. Dies führt zu geringeren Herstellungskosten für das Schlauchsystem, und einer verbesserten Nutzbarkeit. Die verbesserte Nutzbarkeit des Schlauchsystems ist damit zu begründen, dass im Vergleich zu herkömmlichen Systemen weniger Konnektoren verbunden werden müssen, und somit eine Maschine, welche ein derartiges Schlauchsystem verwendet, schneller aufgerüstet werden kann, Leckagen seltener auftreten und das Schlauchsystem übersichtlicher gestaltet ist. Weiterhin hat dieses Schlauchsystem den Vorteil, dass sich ein Schlauchfluid-Luft-Kontakt verringert bzw. vermieden wird. In dem Fall, in dem das Fluid/Flüssigkeit, welches durch den (ersten) Schlauch strömt, Blut ist, verringert sich somit die Gefahr einer Blutgerinnung. Somit müssen dem Blut weniger Gerinnungshemmer zugeführt werden, was Behandlungskosten verringert. Weiterhin entfällt ein Verschleiß maschinenseitiger Druckanschlüsse (Luer-Lock-Anschlüsse), und die Kontaminierungsgefahr aufgrund der Druckmessung ist unterdrückt.

Der hauptsächliche Vorteil des erfindungsgemäßen Verfahrens liegt indessen darin, dass eine Referenzmessung während einer aktiven Verwendung des (ersten) Schlauchs deutlich genauere Werte liefert, als eine alleinige Referenzmessung vor einer aktiven Schlauchverwendung. Die in dem erfindungsgemäßen Verfahren ermittelte Korrekturfunktion lässt sich unabhängig von dem Schlauchmaterial oder den Schlauchabmessungen durchführen und ist daher universell auch für unbekannte Schlauchsysteme verwendbar.

Das Verfahren kann so ausgeführt sein, dass zusätzlich zu dem ersten Druck ein zweiter Druck, der mit einem zweiten Kraftsensor/einem zweiten Drucksensor gemessen wird, der in eine zweite Klemmvorrichtung integriert ist, gemessen und korrigiert werden kann. Dabei wird das Kraftsignal des zweiten Kraftsensors bei der ersten Kalibrierung und bei der zweiten Kalibrierung mit dem Druckreferenzsignal kalibriert, das mit dem zweiten Druckreferenzsensor gemessen wird. Somit können Schlauchinnendrücke an zwei verschiedenen Stellen in einem extrakorporalen Kreislauf ohne einen Fluid-Luft-Kontakt gemessen und korrigiert werden.

Eine vorteilhafte ggf. unabhängig zu beanspruchende Ausführung des erfindungsgemäßen Verfahrens sieht vor, dass der (erste) Schlauch (beispielsweise ein extrakorporaler Blutschlauch einer Dialysemaschine oder einer Blutpumpe) einen arteriellen Abschnitt (Bluteinlassabschnitt) und einen venösen Abschnitt (Blutauslassabschnitt) aufweist. Dabei sind der erste und/oder zweite Druck-/Kraftsensor und der erste Druckreferenzsensor vorzugsweise an dem arteriellen Abschnitt angeordnet. Der zweite Druckreferenzsensor, der ein Druck-/Kraftsensor zur Überprüfung des Schlauchinnendrucks im venösen Abschnitt sein kann, der (als einziger) nicht mit einer Klemmvorrichtung zusammenwirkt, ist am venösen Abschnitt angeordnet. Das heißt, der Druck im venösen Abschnitt des (ersten) Schlauchs wird (als einziger) über den zweiten Druckreferenzsensor bevorzugt nach einem herkömmlichen Druckmessverfahren, bspw. über ein T-Stück oder eine flexible Membran, (mit erhöhter Genauigkeit) gemessen. Das heißt, dass der zweite Druckreferenzsensor (als einziger) weiterhin bevorzugt über einen Luer-Lock-Anschluss mit der Maschine, an welche der (erste) Schlauch angeschlossen ist, verbunden ist. Die Referenzierung erfolgt damit mittels eines Druckreferenzsensors, der im Vergleich zu dem in einer Klemmvorrichtung integrierten Sensor zwar teurer ist aber eine höhere Genauigkeit aufweist. Der erste Druckreferenzsensor ist im Bereich des arteriellen Schlauchabschnitts angeordnet und weist bevorzugt ein piezoelektrisches Element zur Druckmessung auf.

Weiterhin kann vorgesehen sein, dass der konstante Schlauchinnendruck durch das Einstellen eines Pumpverhältnisses zwischen einer ersten Pumpe, insbesondere Blutpumpe, und einer zweiten Pumpe, insbesondere Dialysateingangsflusspumpe oder Dialysatausgangsflusspumpe, erzielt werden kann. Es ist besonders zuverlässig und einfach, einen konstanten Druck im Schlauch über das Einstellen eines Pumpverhältnisses zwischen diesen beiden Pumpen zu erzeugen. Für das erfindungsgemäße Verfahren ist ein konstanter Druck im befüllten Schlauch von wesentlicher Bedeutung.

Weiterhin kann das Verfahren so ausgeführt sein, dass das Driftsignal die Rückstellkraft des geklemmten Schlauchs ist bzw. dieser entspricht.

Darüber hinaus ist vorstellbar, dass das (korrigierte) Kraftsignal mithilfe des entsprechenden Druckreferenzsignals über eine lineare Rekursion in ein Drucksignal umgerechnet wird, bzw. das Kraftsignal mit dem Druckreferenzsignal kalibriert wird. Diese lineare Rekursion ermöglicht eine einfache Berechnung des Drucksignals aus dem jeweiligen Kraftsignal.

Eine weitere, ggf. unabhängig beanspruchbare Ausführungsform sieht vor, dass im Falle von zwei verwendeten Kraftsensoren der erste Kraftsensor an einer Einlauföffnung/einem Bluteinlauf der ersten Pumpe angeordnet, insbesondere integriert, ist und der zweite Kraftsensor an einer Auslauföffnung/einem Blutauslauf der ersten Pumpe angeordnet, insbesondere integriert, ist. Da in diesem Fall das Schlauchmaterial an der Position der Kraftsensoren, die Temperatur im Schlauch und der Einlegezeitpunkt des Schlauchs in die entsprechenden Klemmvorrichtungen identisch sind, sollte das erwartete Driftverhalten an den Positionen der beiden Kraftsensoren ebenfalls identisch sein.

Weiterhin ist eine Vorrichtung vorgesehen, die einen extrakorporalen Kreislauf und zumindest einen Druck-/Kraftsensor, insbesondere arteriellen Druck-/Kraftsensor und/oder Dialysatoreingangs-Druck-/Kraftsensor, der in eine Klemmvorrichtung integriert ist, zur Schlauchinnendruckmessung in einem mit Fluid befüllten Schlauch mit arteriellem und venösem Abschnitt aufweist. Weiterhin weist die Vorrichtung zumindest einen Druckreferenzsensor zum Referenzieren eines Druck-/Kraftsignals auf, welches der zumindest eine Drucksensor ausgibt. Der zumindest eine Druckreferenzsensor, insbesondere ein arterieller und/oder venöser Druckreferenzsensor ist nicht als Klemmvorrichtung ausgeführt bzw. nicht dafür vorgesehen in einer Klemmvorrichtung integriert zu sein (klemmvorrichtungsfreie Ausführung). Weiterhin weist die Vorrichtung bevorzugt zumindest eine erste Pumpe und eine zweite Pumpe auf. Die Vorrichtung ist dazu vorgesehen und angepasst, das Verfahren zur Kalibrierung des Drucksignals des zumindest einen Drucksensors mithilfe eines Referenzsignals des zumindest einen Druckreferenzsensors nach zumindest einem der vorhergehenden Aspekte der Erfindung anzuwenden.

Schließlich ist eine Kalibriervorrichtung zur Kalibrierung der Messung zumindest eines ersten Schlauch-(Innen-)Drucks, vorzugsweise eines arteriellen Drucks, vorgesehen. Dieser erste Druck wird in Form eines Kraftsignals in einem Fluidkreis, insbesondere einem extrakorporalen (Blut-)Kreislauf innerhalb eines mit dem Fluid/Flüssigkeit/Blut befüllten Schlauchs (nicht Bestandteil der Vorrichtung) mithilfe eines am Schlauch (außenseitig) direkt anliegenden oder in Anlage bringbaren ersten Kraftsensors/einem ersten Druck-/Kraftsensor der Kalibriervorrichtung gemessen. Der Druck-/Kraftsensor ist dabei in eine erste Klemmvorrichtung integriert. Der erste Druck wird kalibriert, um ein vom (ersten) Schlauch (Schlauchmaterial) hervorgerufenes Driftsignal durch eine schlauchparameterunabhängige Korrekturfunktion mithilfe eines Druckreferenzsignals, das durch einen ersten Druckreferenzsensor der Kalibriervorrichtung aufgenommen/erzeugt wird, zu korrigieren. Die Kalibriervorrichtung weist folgende Einheiten oder Abschnitte auf:

a) einen ersten Rechnerabschnitt (CPU-Einheit/Programmschritt), vorgesehen und ausgebildet zur Analyse und Vorhersage zumindest einer Korrekturfunktion zur Findung eines Korrektursignals zur Korrektur des Driftsignals mithilfe eines entsprechenden (Kraft-/)Druckreferenzsignals, das durch den ersten Druckreferenzsensor bei konstantem Schlauchinnendruck und konstanter Schlauchinnentemperatur gemessen/erzeugt wird;

b) einen zweiten Rechnerabschnitt (CPU-Einheit/Programmschritt), vorgesehen und ausgebildet zur ersten Kalibrierung des mit dem ersten Druck-/Kraftsensor (PA) gemessenen und dann mithilfe des Korrektursignals korrigierten Kraftsignals mit dem Druckreferenzsignal, das durch den ersten Druckreferenzsensor gemessen/erzeugt wird, vor einer betriebsgerechten Verwendung des Schlauchs; und

c) einen dritten Rechnerabschnitt (CPU-Einheit/Programmschritt), vorgesehen und ausgebildet zur zweiten Kalibrierung des mit dem ersten Druck-/Kraftsensor gemessenen und dann mithilfe des Korrektursignals korrigierten Kraftsignals mit dem (Kraft-/)Druckreferenzsignal, das durch einen zweiten Druckreferenzsensor gemessen/erzeugt wird, während einer aktiven (betriebsgerechten) Verwendung des Schlauchs.

Im Folgenden sind zwei Ausführungsformen des erfindungsgemäßen Verfahrens unter Bezug auf die beigefügten Zeichnungen im Detail beschrieben.

Kurzbeschreibung der Figuren

Fig. 1 zeigt ein Diagramm, das beispielhaft den Druckverlauf in einer Klemmvorrichtung mit der Zeit darstellt;

Fig. 2A zeigt eine Klemmvorrichtung, in die ein Schlauch eingeklemmt ist;

Fig. 2B zeigt ein Diagramm, welches den Verlauf eines Driftsignals im Vergleich mit einem Referenzsignal zeigt;

Fig. 3A zeigt die Front einer Dialysemaschine in einem Zustand vor einer aktiven Verwendung des Schlauchs;

Fig. 3B zeigt eine alternative Anordnung zweier Sensoren;

Fig. 4 zeigt ein Diagramm, welches das Driftverhalten eines Schlauches in einer geschlossenen Klemmvorrichtung zeigt;

Fig. 5 zeigt ein Diagramm, das den Driftsignalverlauf bei einem konstanten Schlauchinnendruck und die das Driftverhalten des Schlauchs abbildende Korrekturfunktion zeigt;

Fig. 6 zeigt ein Diagramm, das graphisch die Bestimmung des Drucksignals aus dem Driftsignal/Kraftsignal zeigt;

Fig. 7 zeigt die Front einer Dialysemaschine bei einer aktiven Verwendung des Schlauchs mit einem an die Maschine angeschlossenen Patienten;

Fig. 8 zeigt ein Diagramm, das beispielhaft den Druckverlauf an einem ersten Drucksensor mit der Zeit bei Durchführung des Verfahrens zeigt;

Fig. 9 zeigt ein Diagramm, das die Druckverläufe eines herkömmlichen Drucksensors, eines ersten Druckreferenzsensors und einer ersten Pumpe zeigt;

Fig. 10A zeigt ein Diagramm, das die Druckverläufe zweier herkömmlicher Drucksensoren und eines zweiten Druckreferenzsensors zeigt, die gleichzeitig mit den Verlaufskurven aus Fig. 10A aufgezeichnet wurden;

Fig. 10B zeigt ein Diagramm, das den zeitlichen Verlauf einer (simulierten) Patientenblutströmungsrate und eines Patientenblutdrucks anzeigt;

Fig. 11A zeigt ein Diagramm, das eine Abweichung zwischen einem errechneten Drucksignal und einem Referenzsignal aufgrund einer Temperaturdrift zeigt:

Fig. 11B zeigt ein Diagramm, das mit dem Diagramm aus Fig. 11A zusammenhängt und den Temperaturverlauf eines Fluids im Schlauch am ersten und zweiten Drucksensor zeigt;

Fig. 12A zeigt ein Diagramm, das die Abweichung des Drucksignals des zweiten Druckreferenzsensors vom entsprechenden Druckreferenzsignal und ein lineares Korrektursignal zeigt;

Fig. 12B zeigt ein Diagramm, das mit dem Diagramm aus Fig. 12A zusammenhängt und die Verläuft des mit dem linearen Korrektursignal korrigierten Drucksignals und des Druckreferenzsignals anzeigt;
Fig. 13A zeigt ein Diagramm, das die Abweichung des Drucksignals des zweiten Druckreferenzsensors vom entsprechenden Druckreferenzsignal und ein polynomisiertes Korrektursignal zeigt;
Fig. 13B zeigt ein Diagramm, das mit dem Diagramm aus Fig. 13A zusammenhängt und die Verläufe des mit dem polynomisierten Korrektursignal korrigierten Drucksignals und des Druckreferenzsignals anzeigt.

Beschreibung der Ausführungsformen

Nachstehend werden Ausführungsformen der vorliegenden Offenbarung auf der Basis der zugehörigen Figuren beschrieben. Es ist anzumerken, dass die dargestellten Werte nur beispielhaft und nicht begrenzend sind.

Erste Ausführungsform

Gesamtverfahren

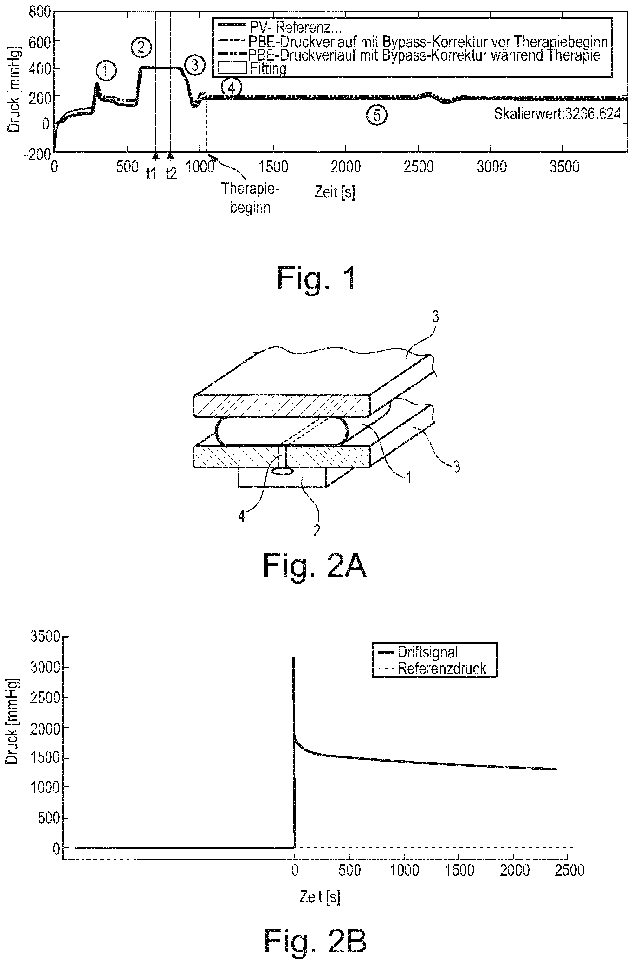

Fig. 1 zeigt beispielhaft den Druckverlauf in Millimeter-Quecksilbersäule (mmHg) eines Drucksensors (hier des PBE-Drucksensors, welcher nachstehend näher erläutert ist) mit der Zeit t in Sekunden (s).
In Phase 1 wird ein Schlauch in zwei Klemmvorrichtungen, in welche jeweils mindestens ein Drucksensor integriert ist und die einen Druck in Form eines Kraftsignals messen, an einer Dialysemaschine eingelegt und der Schlauch mit einem Fluid befüllt. Durch Variation der Flusspumpendrehzahlen zumindest einer Pumpe wird das Schlauchsystem befüllt. In dieser Phase erfolgen außerdem Dichtigkeitstests an Maschine und Schlauch.
In Phase 2 wird der Druck im Schlauch konstant gehalten. Nach einer kurzen Einschwingphase wird der bereits beschriebene Schritt a) zur Regressionsanalyse und Vorhersage zumindest einer Korrekturfunktion zur Findung eines Korrektursignals zur Korrektur des Driftsignals mithilfe eines entsprechenden Druckreferenzsignals, durchgeführt. In Schritt a) wird eine Korrekturfunktion f als Funktion der Zeit t mit zwei Konstanten $a_0$, b für das viskoelastische Verhalten des Schlauchs vor einer Therapie ermittelt. Die zwei Konstanten $a_0$ und b werden mit Hilfe eines mathematischen im Folgenden als "Fitting" bezeichneten Verfahrens ermittelt. Dieses Verfahren wird nachstehend erläutert. Mithilfe dieser Funktion wird ein Driftsignal in Form eines Kraftsignals des jeweiligen Drucksensors bestimmt.
In Phase 3 ist ein Druckabfall zu erkennen, der dazu verwendet wird, das Kraftsignal mithilfe der in Schritt a) ermittelten Korrekturfunktion zu korrigieren. Weiterhin wird das korrigierte Kraftsignal des Drucksensors mit dem dazu vorgesehenen Druckreferenzsignal eines Druckreferenzsensors über einen linearen Zusammenhang zwischen Referenzsignal und korrigiertem Kraftsignal in ein korrigiertes Drucksignal umgerechnet. Dabei ist der Druckreferenzsensor ein herkömmlicher Drucksensor. Phase 3 zeigt somit den Ablauf von Schritt b), der vor dem Beginn der Therapie erfolgt. In Schritt b) wird also das korrigierte Kraftsignal vor der Therapie mit dem Druckreferenzsignal des entsprechenden Druckreferenzsensors kalibriert. Die Kalibrierung kann ebenso über ein zweites konstantes Druckniveau erfolgen.
Als herkömmlicher Drucksensor wird ein Drucksensor bezeichnet, der in keine Klemmvorrichtung integriert ist und bei dem keine Rückstellkraft das Drucksignal beeinflusst. Bei einem herkömmlichen Drucksensor wird ein Schlauchinnendruck beispielsweise über ein T-Stück oder einen Pressure Pod oder ähnliches ermittelt (oben ausgeführt).
In Phase 4 wird nach einer vorbestimmten Zeit nach Therapiebeginn, vorzugsweise nach 5 Minuten, das neu korrigierte Kraftsignal nochmals mit dem Druckreferenzsignal des entsprechenden Druckreferenzsensors kalibriert. In Phase 4 wird somit Schritt c) ausgeführt.
In Phase 5 ist der Therapieverlauf verdeutlicht, während dem der PBE-Druck weitgehend konstant ist. Es ist erkennbar, dass das korrigierte PBE-Drucksignal und das Referenzsignal übereinanderliegen, was heißt, dass die Korrektur des Drucksignals ausreichend ist und auch bei variierendem Druck funktioniert (siehe Zeitintervall zwischen ca. 2600-2700s).
Nachfolgend werden die Klemmvorrichtung, welche als Drucksensor dient, der Aufbau einer Maschine, für welche das erfindungsgemäße Verfahren verwendet wird, sowie die Schritte a) bis c) detailliert und beispielhaft ausgeführt. Außerdem folgen alternative Ausführungsformen der Erfindung.

Hintergrund

Fig. 2A zeigt einen (ersten) Schlauch 1, dessen Innendruck über einen Kraftsensor 2 gemessen werden kann. Dazu ist der Schlauch 1 in eine Klemmvorrichtung 3 eingeklemmt. Diese klemmt den Schlauch 1 ein und eine Ausdehnung oder eine Kontraktion des Schlauchs 1 wird über ein Kraftübertragungsmittel 4 an den Kraftsensor 2 übertragen. Die Kraftänderung, welche der Kraftsensor 2 messen kann, ist proportional zu der Innendruckänderung im Schlauch 1.
Fig. 2B zeigt eine Graphik, welche den Druckverlauf eines Driftsignals und den Druckverlauf eines Referenzsignals mit der Zeit anzeigt. Das dargestellte Drucksignal/Driftsignal zeigt den Verlauf eines Drucks im Schlauch 1, der zum Zeitpunkt t0 in die Klemmvorrichtung 3 eingeklemmt wird. Der Referenzdruck hat über den gesamten dargestellten Zeitverlauf einen Wert von 0mmHg (Umgebungsdruck). Das Drucksignal zeigt zum Zeitpunkt t0 einen Druckanstieg und anschließend einen logarithmisch abnehmenden Druckverlauf, welcher mit der Rückstellkraft des Schlauches begründet werden kann. Dieses Driftsignal muss vom eigentlichen Messsignal abgezogen werden, damit der absolute Druck dem Referenzdruck gleicht.

Aufbau zur Durchführung des erfindungsgemäßen Verfahrens

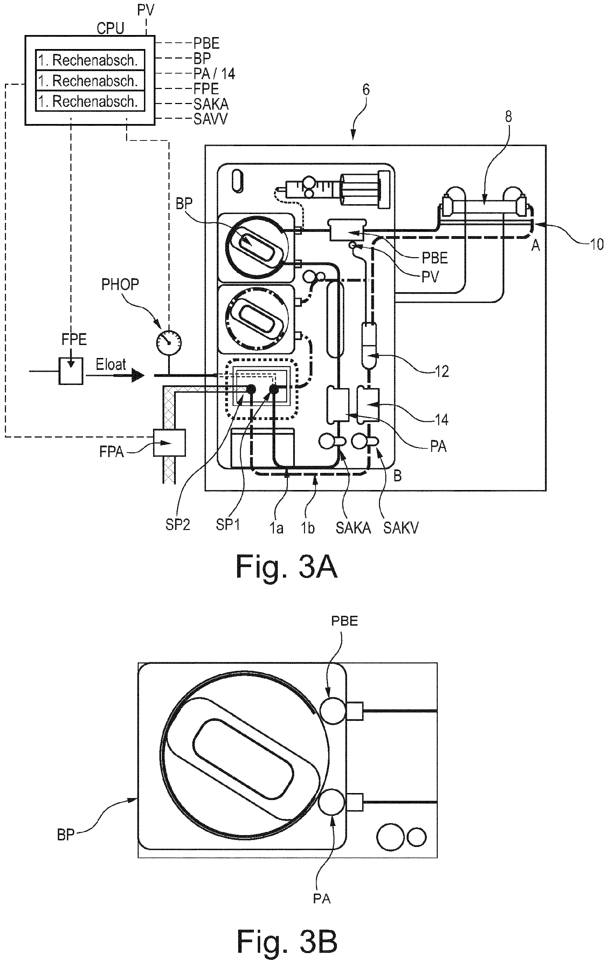

Fig. 3A zeigt die Front einer Dialysemaschine 6, an welcher ein (erster) Schlauch 1 angebracht ist, dessen Innendruck an verschiedenen Stellen gemessen werden soll. Die Dialysemaschine 6 weist einen extrakorporalen Kreislauf auf. Der Schlauch 1 hat einen arteriellen Abschnitt /Zweig 1a und einen venösen Abschnitt 1b. Über einen ersten Substituatport SP1 wird der arterielle Abschnitt 1a des Schlauchs 1 an die Maschine 6 angeschlossen und über einen zweiten Substituatport SP2 wird der venöse Abschnitt 1b des Schlauchs 1 mit der Maschine 6 verbunden. In der dargestellten Ausführungsform ist der Schlauch 1 nicht mit einem Patienten verbunden, das heißt der Schlauch ist nicht in einer aktiven Verwendung und daher in einem Zustand vor einer Therapie. Daher wird der Schlauch 1 hier nicht mit Blut sondern mit einem anderen Fluid befüllt, welches hier ein Substituat (Elektrolytflüssigkeit / Eloat) ist.

Das Fluid wird über eine Dialysateingangsflusspumpe FPE, die außerhalb der Front der Dialysemaschine 6 angeordnet ist, zuerst in den arteriellen Schlauchabschnitt 1a gefördert. Noch bevor das Fluid im Bereich der Front der Dialysemaschine 6 ist, nimmt ein erster Druckreferenzsensor PHOP den Schlauchinnendruck ab, bzw. misst den Schlauchinnendruck. Der Druckreferenzsensor PHOP ist somit ebenfalls am Substituatport SP angeordnet und im Vergleich zu herkömmlichen Dialysemaschinen 6 ein zusätzlicher Drucksensor. Nach dem Eintritt des Fluids in die Front der Dialysemaschine 6 passiert es zuerst die arterielle Schlauchklemme SAKA, welche üblicherweise geöffnet ist. Anschließend passiert das Fluid die erste Klemmvorrichtung, die auch als PA-Drucksensor bzw. erster Drucksensor PA bezeichnet wird, und damit den ersten Kraftsensor. Die erste Klemmvorrichtung ist in die Frontseite der Dialysemaschine 6 integriert. Der PA-Sensor misst den Druck im arteriellen Abschnitt 1a des Schlauches 1. Der Druckreferenzsensor PHOP kann zur (später ausgeführten) Referenzierung des ersten Drucksensors PA herangezogen werden, da er eine höhere Messgenauigkeit als der erste Drucksensor PA aufweist.

Anschließend erreicht das Fluid die erste Pumpe, eine Blutpumpe, BP, welche das Fluid weiter fördert. Schließlich passiert das Fluid eine zweite Klemmvorrichtung, die auch PBE-Drucksensor bzw. zweiter Drucksensor PBE genannt wird, und damit den zweiten Kraftsensor. Der PBE-Drucksensor misst den Dialysatoreingangsdruck an einer Stelle hinter der Blutpumpe BP in Strömungsrichtung des Mediums im Schlauch. Nach dem PBE-Drucksensor kann das Fluid einen Dialysator 8 passieren. Es ist aber im Falle einer Bypassschaltung über den Bypass 10 auch möglich, dass das Fluid nicht den Dialysator durchströmt, sondern diesen umgeht. Hinter dem Dialysator/Bypass in Fluid-Strömungsrichtung liegt der venöse Schlauchabschnitt 1b an. An einer Stelle nach dem Dialysator/Bypass und vor einer Luftfalle 12, in der in das Fluid eingeschlossene Luft aus dem Fluid entfernt wird, passiert das Fluid im venösen Schlauchabschnitt 1b einen herkömmlichen Druckabnehmer, welcher als PV-Messstelle bezeichnet wird. Der herkömmliche Druckabnehmer kann beispielsweise als T-Stück oder als Pressure Pod ausgeführt sein.

Nach der PV-Messstelle passiert das Fluid den Entlüfter 12, anschließend einen Luftdetektor 14 und schließlich eine venöse Schlauchklemme SAKV, welche im Normalfall geöffnet ist. Die venöse und die arterielle Schlauchklemme SAKV, SAKA sind nur in dem Fall geschlossen, in dem ein Fehler vorliegt und sperren bei einer Therapie den Patientenzugang ab. Ein derartiger Fehler kann beispielsweise sein, dass der Luftdetektor eine Menge an Luft detektiert, die größer als ein bestimmter Schwellenwert ist. Nachdem das Fluid die venöse Schlauchklemme SAKV passiert hat, strömt es über den Substituatport SP2 mithilfe einer Pumpleistung einer Dialysatausgangsflusspumpe FPA aus, welche außerhalb der Front der Dialysemaschine 6 angeordnet ist.

Weiterhin zeigt Fig. 3A, dass die Dialysemaschine 6 mit einer CPU verbunden ist, welche einen ersten, einen zweiten und einen dritten Rechnerabschnitt aufweist. Dabei kann die CPU die Dialysateingangsflusspumpe FPE, die Dialysatausgangsflusspumpe FPA, den Druckreferenzsensor PHOP, den Druckreferenzsensor PV, den ersten Drucksensor PA, den zweiten Drucksensor PBE, die Blutpumpe BP, die arterielle Schlauchklemme SAKA und die venöse Schlauchklemme SAKV steuern.

Fig. 3B zeigt die Blutpumpe BP und eine alternative Anordnung der ersten und zweiten Drucksensoren PA und PBE. In diesem Fall befindet sich der erste (arterielle) Drucksensor PA direkt am Bluteinlauf der Blutpumpe BP und der zweite (Dialyatoreingangs-)Drucksensor PBE befindet sich direkt am Blutauslauf der Blutpumpe BP. In diesem Fall sind die beiden Drucksensoren PA und PBE in die Blutpumpe BP integriert und das Schlauchmaterial, die Temperatur und der Einlegezeitpunkt des Schlauchs für beide Drucksensoren PA, PBE sind identisch. Auch das erwartbare Driftverhalten ist für beide Drucksensoren PA, PBE identisch. Somit kann der Differenzdruck $P_{PBE} - P_A$ beider Drucksensoren ohne Korrektur der Drift, das heißt ohne Kalibrierung, bestimmt werden. Der Differenzdruck $P_{PBE} - P_A$ sollte während der gesamten Therapie der Differenz der korrigierten Drücke $P_{PBE\_korr} - P_{A\_korr}$ entsprechen. Durch Vergleichen der beiden Differenzdrücke (unkorrigierte mit korrigierter Differenz) kann die Richtigkeit der Korrekturfunktion, die später beschrieben ist, beurteilt werden. Bei einer Abweichung der Differenzdrücke voneinander um mehr als einen vorbestimmten Betrag ist eine Rekalibrierung des Systems sinnvoll.

Schritt a)

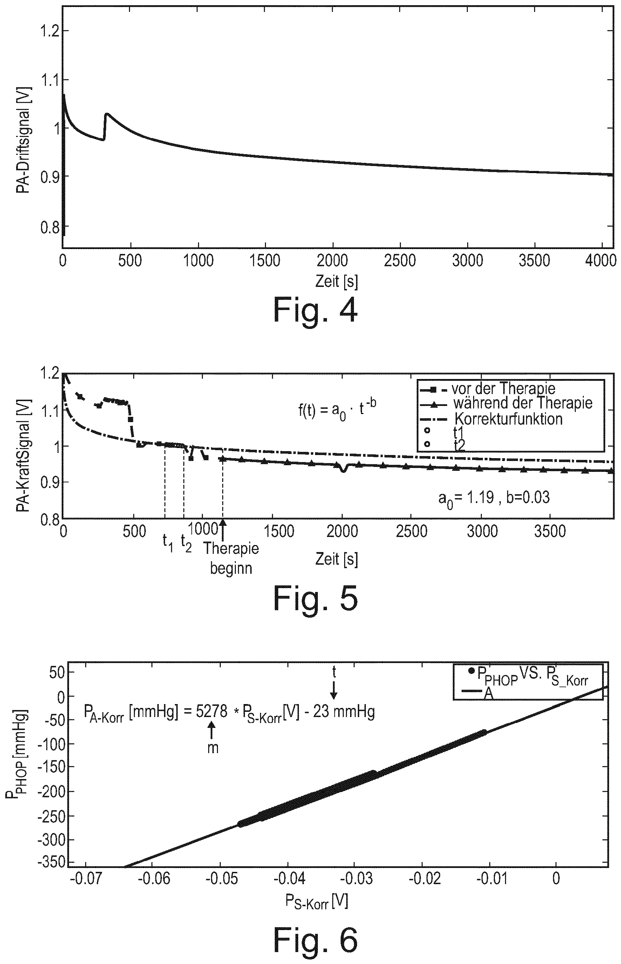

Ähnlich wie Fig. 2B zeigt Fig. 4 den Signalverlauf des eingeklemmten Schlauchs nach dem Schließen der Klemmvorrichtung. Allerdings ist hier beispielhaft das Kraftsignal des ersten Drucksensors PA in Form von Spannungswerten in der Einheit Volt (V) im Verlauf mit der Zeit t in Sekunden (s) dargestellt. Zum Zeitpunkt t0 (t0=0s) wird die Klemmvorrichtung geschlossen. Es zeigt sich der bereits aus Fig. 2B bekannte Signalverlauf für eine Zeitspanne vor und während der Therapie. Die Abnahme des Signalverlaufs ist mit den viskoelastischen Eigenschaften des Schlauchmaterials zu begründen, welches die Druckübertragung zwischen dem Kraftsensor und dem Fluid im Schlauch beeinflusst. Der elastische Anteil des Schlauchs erzeugt eine Rückstellkraft. Der viskose Anteil des Schlauchs führt zu einer langsamen, irreversiblen Deformation des Schlauchs. Durch diese Schlauchdeformation nimmt die Rückstellkraft ab und damit auch die Kraft, mit der der Schlauch auf den Kraftsensor drückt. Der Verlauf der Rückstellkraft, die in Fig. 4 dargestellt ist, wird auch als Driftsignal bezeichnet. Um das Kraftsignal bzw. Drucksignal des Kraftsensors als Signal darstellen zu können, das nur vom Schlauchinnendruck abhängt, ist es sinnvoll, das Driftsignal zu bestimmen, um es dann aus dem gemessenen Kraftsignal bzw. Drucksignal herausrechnen/eliminieren zu können, d.h. das Driftsignal von dem gemessenen Kraftsignal zu subtrahieren.

Das viskoelastische Verhalten des Schlauches folgt allgemein der Gleichung (1), sodass das Driftsignal als mathematische Korrekturfunktion beschrieben werden kann, welche damit folgender Gleichung folgt:

$$f(t) = a_0 \cdot t^{-b} \qquad\qquad (1)$$

Dabei ist t die Zeit und $a_0$ und b sind unbekannte Konstanten. f(t) hat die Einheit V, da das Kraftsignal als Spannungswert ausgegeben wird.

Zur weiteren Verwendung der Gleichung ist es sinnvoll, die Konstanten $a_0$ und b durch Fitting zu bestimmen. Dazu ist es durch Einstellen des Pumpverhältnisses der Pumpen BP und FPE oder FPA erforderlich, einen konstanten Schlauchinnendruck zu erzeugen. Weiterhin ist eine konstante Schlauchinnentemperatur erforderlich. Das Substituat, welches durch den Schlauch strömt, ist auf 36° vorgewärmt, sodass auch die Schlauchinnentemperatur konstant ist. Weiterhin wird das Kraftsignal eines Drucksensors, hier des ersten Drucksensors PA, in einer Messung ermittelt. Allerdings kann auch das Kraftsignal eines anderen Drucksensors, wie beispielsweise des zweiten Drucksensors PBE, verwendet werden. Der Verlauf des Kraftsignals des ersten Drucksensors PA mit der Zeit ist Fig. 5 zu entnehmen.

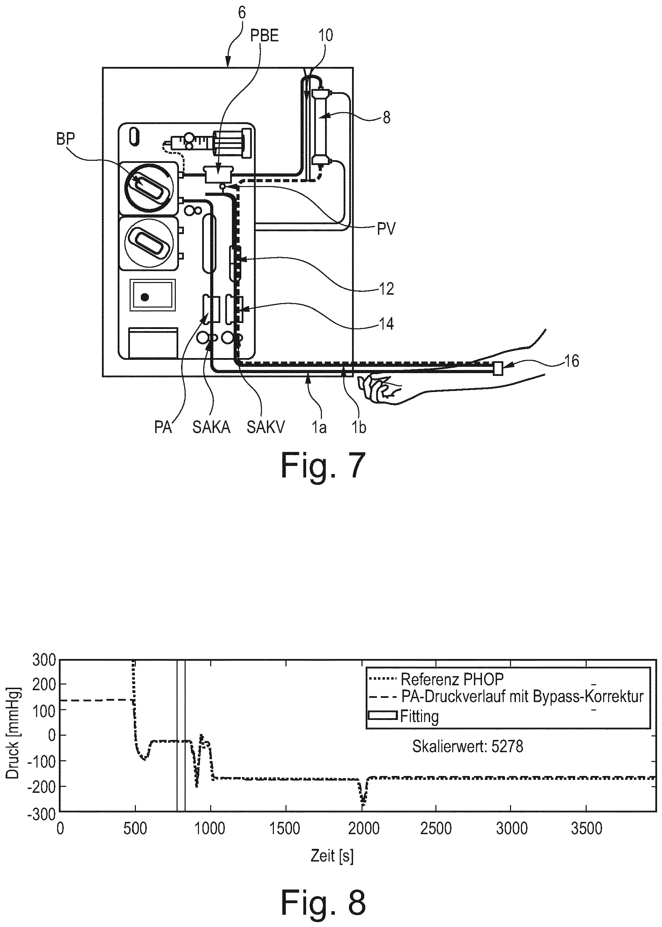

In der Fig. 5 ist das Kraftsignal als Spannung mit der Einheit Volt [V] als Funktion der Zeit t in Sekunden [s] dargestellt. Im Zeitbereich von 0s bis ca. 1200s, während des sogenannten "Primings" liegt ein Zustand vor einer aktiven Verwendung des Schlauchs vor, das heißt vor einer Therapie. Vor der Therapie ist kein Patient mit der Dialysemaschine 6 verbunden und die Dialysemaschine 6 ist in diesem Fall so ausgeführt, wie es im Zusammenhang mit Fig. 3A beschrieben und gezeigt ist. Im Zeitbereich ab ca. 1200s ist das Spannungssignal während der Therapie gezeigt, auch "Therapy" genannt. Während der Therapie ist ein Patient an die Dialysemaschine 6 angeschlossen, welche dann so ausgeführt ist, wie es in Zusammenhang mit Fig. 7 beschrieben und gezeigt ist.

Um die Konstanten $a_0$ und b mithilfe dieses Kraftsignals bestimmen zu können, werden im Fitting zu zwei konkreten Zeitpunkten t1 und t2 im Bereich vor der Therapie die jeweiligen Signalwerte des Kraftsignals f(t=t1) und f(t=t2) ermittelt. Im Beispiel, das in Fig. 5 gezeigt ist, wurden die Zeitpunkte t1 = 600s und t2 = 800s gewählt. Es ergibt sich das folgende Gleichungssystem als Formeln (2) und (3), welches gelöst werden muss, um die Konstanten $a_0$ und b zu erhalten:

$$\text{I} \qquad f(t=t1) = a_0 \cdot t1^{-b} \qquad\qquad (2)$$

$$\text{II} \qquad f(t=t2) = a_0 \cdot t2^{-b} \qquad\qquad (3)$$

Aus Gleichung I, d.h. Formel (2), lässt sich $a_0$ folgendermaßen darstellen:

$$a_0 = f(t=t1) \cdot t1^{b} \qquad\qquad (4)$$

Durch Einsetzen von a0 in der Form, wie es in Formel (4) dargestellt ist, in die Formel (3) ergibt sich b in der in Formel (5) dargestellten Form, in der es nun nur noch von den bekannten Zeitpunkten t1, t2 und den entsprechenden Signalwerten des Kraftsignals f(t=t1) und f(t=t2) abhängt und somit berechnet werden kann:

$$b = \ln\,(f(t=t2)) - \ln\,(f(t=t1))\,/\,(\ln(t1) - \ln(t2)) \tag{5}$$

Nach der Bestimmung des Wertes von b kann dieser Wert in Formel (4) eingesetzt werden, sodass sich der Wert der Konstanten $a_0$ ergibt, und die Gleichung (1) das viskoelastische Verhalten des verwendeten Schlauchs darstellt. Mit den oben gewählten Zeitpunkten t1 und t2 und den in diesem Versuch geltenden Spannungswerten ergibt sich für $a_0$ ein Wert von 1,19 und für b ein Wert von 0,03.

Schritt a) ist hier beispielhaft für den PA-Drucksensor ausgeführt und erfolgt analog dazu für den PBE-Drucksensor.

Schritt b)

Als Nächstes soll das gemessene Kraftsignal korrigiert werden und mithilfe des geeigneten Druckreferenzsignals in ein Drucksignal umgerechnet werden. Dies geschieht in einer Phase vor der Therapie und bei sich änderndem, beispielsweise bei abfallendem, Schlauchinnendruck, oder über ein zweites konstantes Druckniveau mit in Vergleich zum ersten Niveau unterschiedlichen Druck. Der Schlauchinnendruck fällt beispielsweise bei einem Abstecken des Schlauchs von den Substituatports SP1, SP2 zur Vorbereitung einer Therapie ab. Ein zweites Druckniveau kann über ein abweichendes Pumpverhältnis von Blutpumpe und Flusspumpe eingestellt werden. Schritt b) ist beispielhaft für den ersten Drucksensor PA ausgeführt.

Zuerst wird von dem mit dem ersten Drucksensor PA gemessenen Kraftsignal $P_{S\_gem}$, welches als Spannungswert ausgegeben wird (in V), das Driftsignal f(t) mit den berechneten Werten für $a_0$ und b subtrahiert. Damit folgt das korrigierte Kraftsignal $P_{S\_Korr}$ folgender Gleichung (Formel (6)):

$$P_{S\_Korr} = P_{S\_gem} - f(t) \tag{6}$$

Dann werden mit dem ersten Druckreferenzsensor PHOP Druckreferenzwerte $P_{PHOP}$ in der Einheit mmHg aufgenommen und über dem damit jeweils zusammenhängenden Spannungswert $P_{S\_Korr}$ in V in einem Diagramm aufgetragen, welches beispielhaft in Fig. 6 gezeigt ist.

Im Diagramm aus Fig. 6 sind die gemessenen Druckreferenzwerte $P_{PHOP}$ auf der y-Achse über den berechneten Spannungswerten $P_{S\_korr}$ auf der x-Achse als Punkte aufgetragen und für diese Punkte zeigt sich ein linearer Verlauf. Passend dazu wird mathematisch eine Gerade A bestimmt, welche am besten durch diese Punkte verläuft und damit den Zusammenhang zwischen Spannungswerten $P_{S\_korr}$ und den daraus berechneten Druckkorrekturwerten $P_{A\_Korr}$ darstellt. Dieser Zusammenhang kann mathematisch folgendermaßen in Form von Formel (7) angegeben werden:

$$P_{A\_Korr} = m \cdot P_{S\_korr} + t \tag{7}$$

Dabei ist m die Steigung der Gerade, welche auch Skalierungswert genannt wird, und t der Druckreferenzwert, bei welchem die Gerade die y-Achse schneidet und welcher auch als Offsetwert bezeichnet wird. In dem dargestellten Beispiel ergibt sich für den Skalierungswert ein Wert von 5278 mmHg/V und für den Offsetwert ein Wert von 23mmHg.

Damit ist das korrigierte und damit korrekte Drucksignal $P_{A\_korr}$ der ersten Klemmvorrichtung, d.h. des ersten Drucksensors PA, vor der Therapie bekannt und Schritt b) abgeschlossen.

Schritt b) erfolgt für den PBE-Drucksensor analog zu dem hier dargestellten Ablauf für den PA-Drucksensor, allerdings wird hier nicht der PHOP-Druckreferenzsensor als Referenz verwendet, sondern der PV-Druckreferenzsensor.

Da sich beispielsweise die Schlauchinnendruckwerte und/oder die Schlauchinnentemperaturen von einem Zustand vor der Therapie zu einem Zustand während der Therapie ändern können, ist es empfehlenswert, die Korrektur für das gemessene Kraftsignal des ersten und/oder zweiten Drucksensors wiederholt während der Therapie durchzuführen.

Schritt c)

Während der Therapie ändert sich der Aufbau, der in Fig. 2A gezeigt ist, so ab, wie es in Fig. 7 gezeigt ist. In Fig. 7 ist zu erkennen, dass der arterielle und der venöse Schlauchabschnitt 1a und 1b mit dem Patienten verbunden sind. Das Patientenherz ersetzt in diesem Fall die Dialysateingangs- und Dialysatausgangsflusspumpen. Eine (Arm)-arterie, welche mit dem arteriellen Schlauchabschnitt 1a verbunden ist, und eine (Arm)-vene des Patienten, welche mit dem venösen Schlauchabschnitt 1b verbunden ist, sind über eine künstliche Verbindung 16, insbesondere über einen Patientenshunt, miteinander verbunden. Dadurch liegen in Vene und Arterie (können verallgemeinert auch als Adern bezeichnet werden) des Patienten derselbe Blutdruck und dieselben Blutflusswerte vor. Durch Einstellen eines Bypasses am Dialysator liegen auch im arteriellen und im venösen Schlauchabschnitt 1a, 1b und damit im gesamten System, bestehend aus Schlauch und Patientenadern, dieselben Blutdrücke und Blutflusswerte vor. Für Versuche ist es denkbar, einen experimentellen Patientenkreislauf zu simulieren, welcher eine Wasserpumpe, ein beheiztes Wasserbad und ein Gegendruckventil aufweist.

Das Prinzip zur Kalibrierung und Referenzierung ist für den Schritt c) genauso wie für den Schritt b). Erneut wird das gemessene Kraftsignal des Drucksensors durch das unter Schritt a) gefundene Korrektursignal korrigiert und aus dem aus Schritt b) bekannten Zusammenhang zwischen korrigiertem Drucksignal und korrigiertem Kraftsignal (vgl. Formel (6) mit dem in Schritt b) bestimmten Skalierungswert und Offsetwert) kann das korrigierte Drucksignal berechnet werden.

Schritt c) kann für den ersten und den zweiten Drucksensor PA, PBE durchgeführt werden. Allerdings dient hier der konventionelle PV-Druckreferenzsensor sowohl für den PBE-Drucksensor als auch für den PA-Drucksensor als Druckreferenzsensor, um eine gleichzeitige Referenzierung des PA-Drucksignals und des PBE-Drucksignals zu ermöglichen.

Es ist vereinfachend dazu möglich, allein das Drucksignal des zweiten Drucksensors PBE mithilfe des Druckreferenzsensors PV zu referenzieren, indem der Dialysatorfluss in den Bypass geschaltet ist. Es ist aufgrund der herstellungsbedingt gleichen Eigenschaften des Schlauchs an Position der PA-Klemmvorrichtung und an Position der PBE-Klemmvorrichtung die für das PBE-Drucksignal gefundene Korrekturfunktion auf das PA-Drucksignal anzuwenden, wenn diese Methode auch nicht so genau ist, wie jeweils eine Korrekturfunktion für das PA-Drucksignal und das PBE-Drucksignal zu ermitteln.

Ergebnis des erfindungsgemäßen Verfahrens

Nach Filtern und Skalieren des korrigierten Drucksignals kann dieses Signal mit dem entsprechenden, direkt gemessenen Druckreferenzsignal verglichen werden. Dieser Vergleich ist in Fig. 8 beispielhaft für das korrigierte Drucksignal $P_{A\_korr}$ und das entsprechende Druckreferenzsignal $P_{PHOP}$ dargestellt.

In dem Diagramm in Fig. 8 ist zu erkennen, dass der Verlauf des berechneten Druckkorrektursignals $P_{A\_Korr}$ und der Verlauf des direkt gemessenen Druckreferenzsignal $P_{PHOP}$ deckungsgleich sind. Das heißt, dass die mathematische Korrekturfunktion aus (1) zusammen mit den errechneten Werten für $a_0$ und b den Driftverlauf eliminieren kann, was aber nur dann funktioniert, wenn der Schlauchinnendruck und die Schlauchinnentemperatur konstant sind. Falls beispielsweise der Schlauchinnendruck beim Abgreifen des zweiten Spannungswertes f(t2) variiert, ist die Korrekturfunktion nicht für die Beschreibung des Driftsignalverlaufs geeignet.

Referenzierbarkeit

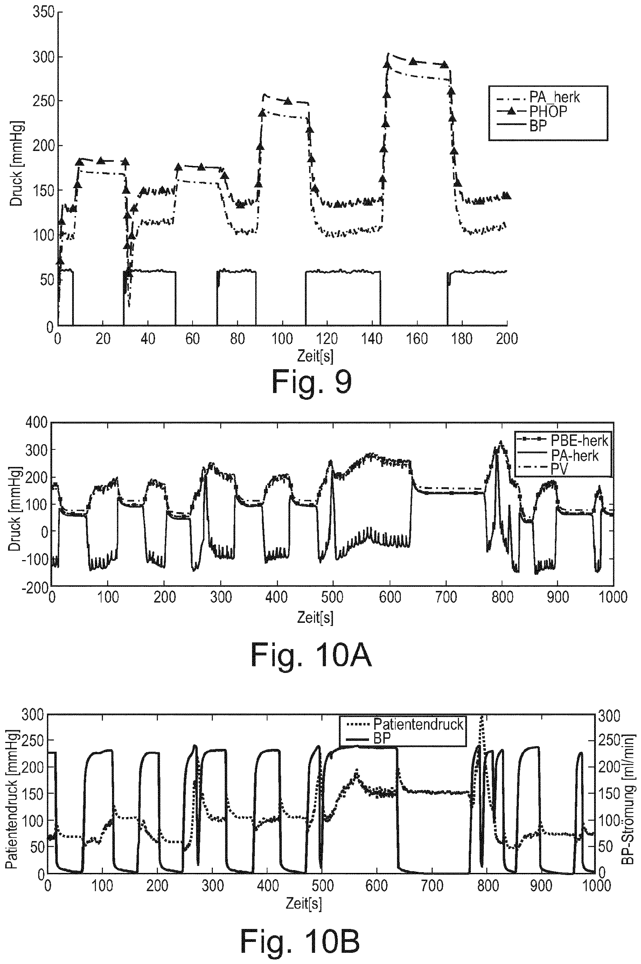

Nachfolgend zeigen Fig. 9 und Fig. 10A die Druckverläufe der herkömmlich verwendeten Drucksensoren zur Messung des arteriellen Drucks $P_A$ und des Dialysatoreinangsdrucks $P_{PBE}$ im Vergleich mit dem Druckverlauf des entsprechenden Druckreferenzsensors PHOP bzw. PV.

In Fig. 9 wird der Druckverlauf eines herkömmlichen ersten Drucksensors PA_herk zur Messung des arteriellen Drucks mit dem Druckverlauf des ersten Druckreferenzsensors PHOP vor der Therapie verglichen. Die Druckverläufe in mmHg sind im Verlauf mit der Zeit t in s dargestellt. Weiterhin ist der Druckverlauf der Blutpumpe BP dargestellt, welcher wiederholt einen Druck von 0 aufweist. Die Blutpumpe BP wird wiederkehrend gestoppt, um konstante Druckwerte des PA_herk-Sensors und des PHOP-Sensors zu erzeugen.

Es ist zu erkennen, dass die Verläufe der beiden Drucksignale des PA_herk-Drucksensors und des PHOP-Druckreferenzsensors gleichartig sind und parallel zueinander verlaufen, hier mit einer Parallelverschiebung der Kurven/einer Druckdifferenz von ca. 20 mmHg. Der Unterschied zwischen den beiden Drucksignalverläufen liegt an den Höhenunterschieden des ersten Drucksensors PA_herk und des ersten Druckreferenzsensors PHOP. Im vorliegenden Ausführungsbeispiel ist der erste Druckreferenzsensor PHOP höher angebracht als der erste Drucksensor PA (siehe Fig. 2A).

Die Druckdifferenz zwischen herkömmlichem Drucksensor und Druckreferenzsensor ist zwar zu beachten, aber der Vergleich in Fig. 9 zeigt, dass der Druckreferenzsensor als Referenzsensor für den arteriellen Druck geeignet ist.

In Fig. 10A sind die Druckverläufe des ersten herkömmlichen Drucksensors PA_herk zur Messung des arteriellen Drucks und eines zweiten herkömmlichen Drucksensors PBE_herk zur Messung des Dialysatoreinangsdrucks im Vergleich mit dem Druckverlauf des zweiten Druckreferenzsensors PV in mmHg mit der Zeit t in s während der Therapie dargestellt. Fig. 10B zeigt ein Diagramm, in dem die zeitlichen Verläufe des Blutpumpenflusses in ml/min und des simulierten Patientendrucks in mmHg dargestellt sind, welche gleichzeitig mit den Druckwerten aus Fig. 10A aufgezeichnet wurden.

In den Bereichen, in denen der Blutpumpenfluss 0 ist, d.h. zu den Zeiten, zu denen die Blutpumpe gestoppt ist, gleichen sich die Drucksignale der Sensoren PA_herk, PBE_herk und PV aneinander an und sind konstant. In diesen konstanten Druckbereichen sind die Druckverläufe der beiden Drucksensoren PA_herk und PBE_herk im Wesentlichen deckungsgleich und es existiert eine Druckdifferenz zu dem Druckverlauf des Druckreferenzsensors PV, die hier etwa 20 mmHg beträgt, und mit dem Höhenunterschied zwischen den Drucksensoren PA_herk, PBE_herk und dem Druckreferenzsensor PV begründet werden kann.

Auch hier gilt, dass die Druckdifferenz zwischen herkömmlichem Drucksensor und Druckreferenzsensor zwar zu beachten ist, aber der Vergleich in Fig. 9 zeigt, dass der zweite Druckreferenzsensor als Referenzsensor für den arteriellen Druck und den Dialysatoreingangsdruck während der Therapie geeignet ist.

Temperaturdrift

Bisher wurde in der obigen Beschreibung von konstantem Schlauchinnendruck und konstanter Schlauchinnentemperatur ausgegangen. Allerdings kann es zwischen Schritt b) und Schritt c), also zwischen der Phase vor der Therapie und der Phase während der Therapie zu Temperaturunterschieden im befüllten Schlauch kommen, welche zu linearen Abweichungen zwischen Referenzsensorwert und Drucksensorwert führen können.

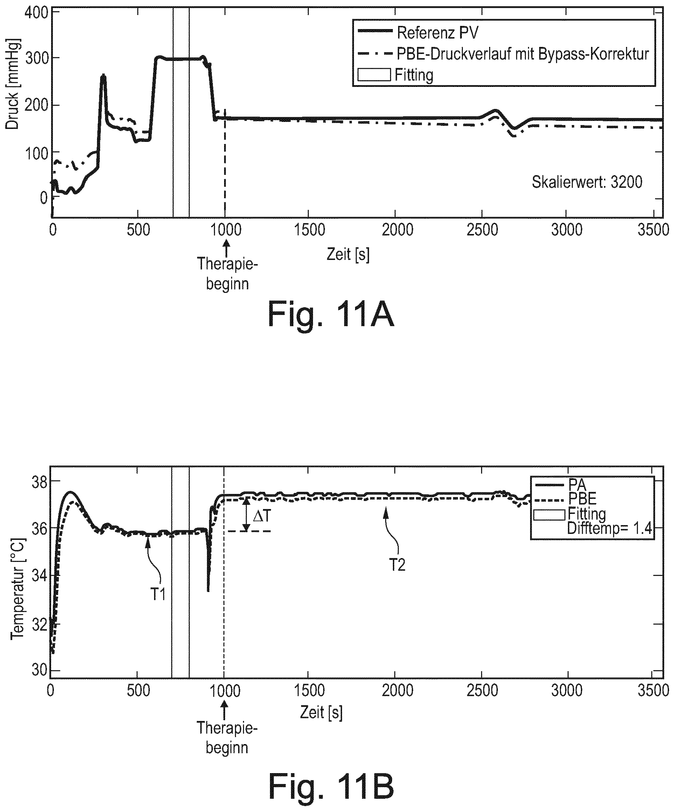

Eine solche Abweichung ist beispielhaft für den PBE-Drucksensor und den PV-Druckreferenzsensor im Diagramm aus Fig. 11A dargestellt. Fig. 11B zeigt den dazugehörigen Temperaturverlauf an den Messstellen des PBE-Drucksensors und des PV-Druckreferenzsensors. Um die Temperatur an diesen beiden Messstellen bestimmen zu können, ist eine Integration eines Temperatursensors in die PBE-Klemmvorrichtung und/oder die PA-Klemmvorrichtung erforderlich. Die Kalibrierung vor der Therapie (bis ca. 900 s) erfolgt hier bei T1, beispielsweise bei 35,8 °C, während die Kalibrierung während der Therapie bei T2, beispielsweise bei 37,2 °C erfolgt. Aufgrund des Temperaturunterschiedes $\Delta T$ (=T2 - T1) vor und während der Therapie, der hier 1,4 °C beträgt, folgt das errechnete PBE-Drucksignal, das in Fig. 11Bdargestellt ist, während der Therapie nicht dem PV-Referenzsignal, sondern weicht linear davon ab. Untersuchungen haben ergeben, dass sich die Abweichung zwischen Drucksignal und Referenzsignal linear proportional zu der Temperaturabweichung vor und während der Therapie verhält. Mithilfe einer empirisch ermittelten Korrekturfunktion kann das Drucksignal korrigiert werden.

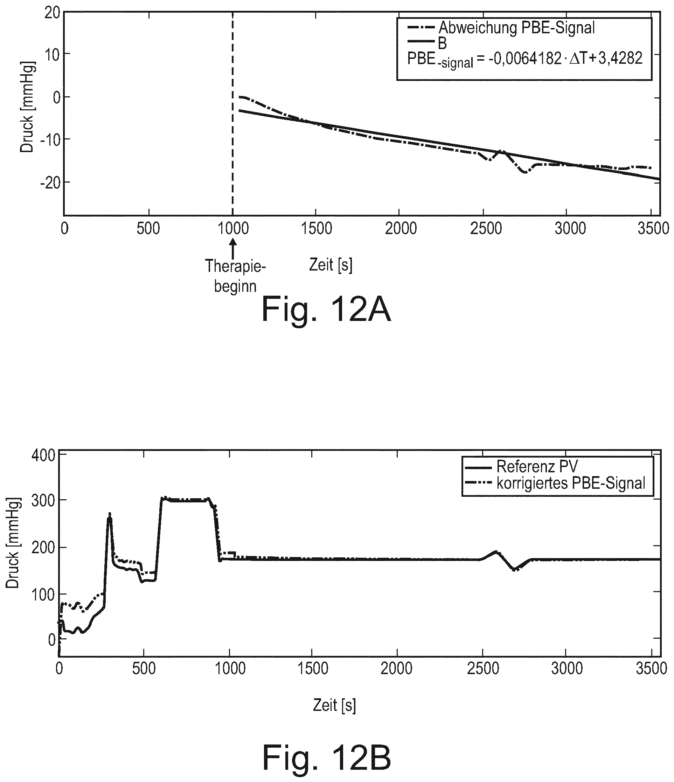

Fig. 12A zeigt die Druckabweichung zwischen Drucksignal und Referenzsignal und eine dafür gefundene Gerade B über der Zeit t in s. Im Beispiel hat die Geradengleichung folgende Form, die in Formel (8) gezeigt ist:

$$PBE_{Signal} = -0,0064182 \cdot \Delta T + 3,4282 \tag{8}$$

Fig. 12B zeigt das mithilfe der bestimmten Formel (8) korrigierte PBE-Drucksignal, das nun wieder deckungsgleich mit dem PV-Referenzsignal dargestellt ist.

Alternativ zu einem linearen Zusammenhang zwischen der Abweichung zwischen Drucksignal und Referenzsignal zu der Temperaturabweichung vor und während der Therapie kann auch ein polynomialer Zusammenhang bestehen. Zur Berechnung der entsprechenden Formel wird allerdings mehr Rechenleistung benötigt, auch wenn ein derartiger Zusammenhang die Abweichung genauer darstellen kann als ein linearer.

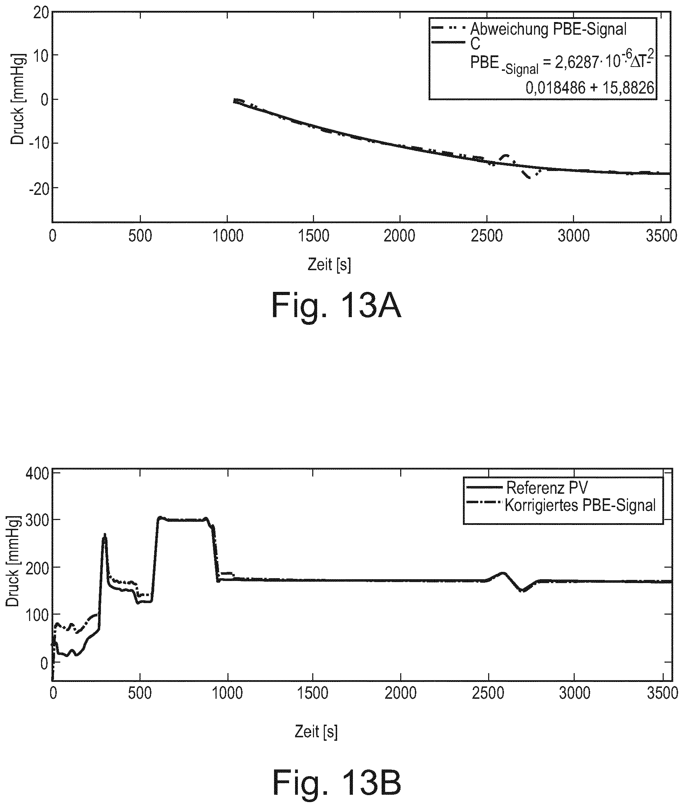

Fig. 13A zeigt, wie auch Fig. 12A, die Druckabweichung zwischen Drucksignal und Referenzsignal und ein dafür errechnetes Polynom C über der Zeit t in Sekunden. Im Beispiel folgt das PBE-Drucksignal folgender polynomialen Abweichung, die in Formel (9) gezeigt ist:

$$PBE_{Signal} = 2,6287 \cdot 10^{-6} \cdot \Delta T^2 - 0,018486 + 15,8826 \tag{9}$$

Fig. 13A zeigt das mithilfe der bestimmten Formel (9) korrigierte PBE-Drucksignal, das nun wieder deckungsgleich mit dem PV-Referenzsignal dargestellt ist.

Zweite Ausführungsform

Die zweite Ausführungsform ähnelt der ersten Ausführungsform, weshalb nachfolgend nur die Unterschiede zur ersten Ausführungsform ausgeführt werden.

Die Referenzdruckmessung der Drucksignale PBE und PA in Schritt c) (während der Therapie) kann alternativ zu der in der ersten Ausführungsform beschriebenen Methode auch mithilfe der venösen Schlauchklemme SAKV und der arteriellen Schlauchklemme SAKA erfolgen.

Dazu wird der Dialysatorfluss, wie auch im ersten Ausführungsbeispiel, in den Bypass geschaltet und die Schlauchklemmen SAKV und SAKA werden geschlossen. Es entsteht eine druckdichte Verbindung im Schlauch. Die Blutpumpe BP wird gestoppt. Allerdings rotiert die Blutpumpe BP verzögerungsbedingt auch nach dem Stopp für kurze Zeit weiter, sodass sich auf im arteriellen Schlauchabschnitt ein negativer Druck und im venösen Schlauchabschnitt ein positiver Druck aufbaut, welche über die Zeit im selben Druckverhältnis zueinander stehen. Das PBE-Drucksignal wird mithilfe des PV-Druckreferenzsensors kalibriert. Auch das PA-Drucksignal kann mithilfe des PV-Druckreferenzsensors kalibriert werden.

Da der Patient aufgrund der geschlossenen Schlauchklemmen SAKV und SAKA vom extrakorporalen Kreislauf abgeschnitten ist, ist diese alternative Ausführung im Schritt c) patientenunabhängig durchführbar. Allerdings zirkuliert das Blut in diesem Fall im extrakorporalen Kreislauf nicht mehr, sodass das Blut im Kreislauf gerinnen kann und die Temperatur absinken kann. Allerdings sind die Gerinnung des Blutes und dessen Temperaturabfall von der Dauer des Blutpumpenstopps abhängig, der daher so kurz wie möglich sein sollte.

**Patentansprüche**

1. Verfahren zur Kalibrierung eines ersten Druck- oder Kraftsensors (PA), der einen ersten Druck einer Flüssigkeit, die innerhalb eines Schlauchs (1), der ein Dialysatorschlauch in einem extrakorporalen Kreislauf ist, vorliegt, in Form eines Kraftsignals misst, wobei der erste Druck- oder Kraftsensor (PA) direkt am Schlauch (1) anliegt und in eine erste Klemmvorrichtung integriert oder in diese eingesetzt ist, **gekennzeichnet durch** die folgenden Schritte:

   a) Regressionsanalyse und Vorhersage zumindest einer schlauchparameterunabhängigen Korrekturfunktion zur Findung eines Korrektursignals zur Korrektur eines durch das Einklemmen des Schlauchs in die erste Klemmvorrichtung hervorgerufenen Driftsignals mithilfe eines entsprechenden Kraft- oder Druckreferenzsignals, das durch einen ersten Kraft- oder Druckreferenzsensor (PHOP) bei einem konstanten Schlauchinnendruck und einer konstanten Schlauchinnentemperatur gemessen wird;
   b) erstes Kalibrieren des mit dem ersten Druck- oder Kraftsensor (PA) gemessenen und mithilfe des Korrektursignals korrigierten Druck- oder Kraftsignals mit dem Kraft- oder Druckreferenzsignal, das durch den ersten Kraft- oder Druckreferenzsensor (PHOP) gemessen wird, vor einer aktiven Verwendung des Schlauchs, bei der der Schlauch an einen Patienten angeschlossen ist; und
   c) zweites Kalibrieren des mit dem ersten Druck- oder Kraftsensor (PA) gemessenen und mithilfe der Korrekturfunktion korrigierten Druck- oder Kraftsignals mit dem Kraft- oder Druckreferenzsignal, das durch einen zweiten Kraft- oder Druckreferenzsensor (PV) gemessen wird, während einer aktiven Verwendung des Schlauchs.

2. Verfahren zur Kalibrierung nach Anspruch 1, **dadurch gekennzeichnet, dass** zusätzlich zu dem ersten Druck ein zweiter Druck, insbesondere Dialyatoreingangsdruck, der mit einem zweiten Kraft- oder Drucksensor (PBE) gemessen wird, der in eine zweite Klemmvorrichtung integriert ist, gemessen und korrigiert wird; und
   das Druck- oder Kraftsignal des zweiten Druck- oder Kraftsensors (PBE) bei der ersten Kalibration und bei der zweiten Kalibration mit dem Kraft- oder Druckreferenzsignal kalibriert wird, das mit dem zweiten Kraft- oder Druckreferenzsensor (PV) gemessen wird.

3. Verfahren zur Kalibrierung nach Anspruch 1, **dadurch gekennzeichnet, dass** der Schlauch (1) einen arteriellen Abschnitt (1a) und einen venösen Abschnitt (1b) aufweist und der erste und/oder zweite Druck- oder Kraftsensor (PA, PBE) und, im Fall vor einer aktiven Verwendung des Schlauchs (1) der erste Kraft- oder Druckreferenzsensor (PHOP), an dem arteriellen Abschnitt (1a) angeordnet werden oder sind, während der zweite Kraft- oder Druckreferenzsensor (PV), der nicht mit einer Klemmvorrichtung zusammenwirkt, am venösen Abschnitt (1b) angeordnet wird oder ist.

4. Verfahren zur Kalibrierung nach Anspruch 1, **dadurch gekennzeichnet, dass** der erste und der zweite Kraft- oder Druckreferenzsensor (PHOP, PV) jeweils eine höhere Messgenauigkeit als der entsprechende Drucksensor (PA, PBE) aufweist.

5. Verfahren zur Kalibrierung nach Anspruch 2, **dadurch gekennzeichnet, dass** zur Kalibrierung des zweiten Kraft- oder Drucksensors (PBE) und zur Kalibrierung des ersten und des zweiten Kraft- oder Drucksensors (PA, PBE) der Schlauchinnendruck durch eine Bypassschaltung in dem arteriellen Abschnitt (1a) des Schlauchs (1) dem Schlauchinnendruck in dem venösen Abschnitt (1b) des Schlauchs (1) angeglichen wird.

**6.** Verfahren zur Kalibrierung nach Anspruch 1, **dadurch gekennzeichnet, dass** der konstante Schlauchinnendruck vor einer aktiven Verwendung des Schlauchs durch das Einstellen eines Pumpverhältnisses zwischen einer ersten Pumpe, insbesondere Blutpumpe (BP), und einer zweiten Pumpe, insbesondere Dialysateingangsflusspumpe (FPE) und/oder Dialysatausgangsflusspumpe (FPA), erzielt wird.

**7.** Verfahren zur Kalibrierung nach Anspruch 1, **dadurch gekennzeichnet, dass** das Driftsignal die Rückstellkraft des geklemmten Schlauchs (1) ist oder dieser entspricht.

**8.** Verfahren zur Kalibrierung nach Anspruch 1, **dadurch gekennzeichnet, dass** das erste und/oder zweite Druck- oder Kraftsignal mithilfe des entsprechenden Kraft- oder Druckreferenzsignals über eine lineare Rekursion in ein Drucksignal umgerechnet wird.

**9.** Verfahren zur Kalibrierung nach Anspruch 4, **dadurch gekennzeichnet, dass** im Falle von zwei verwendeten Druck- oder Kraftsensoren der erste Druck- oder Kraftsensor (PA) an einer Einlauföffnung der ersten Pumpe angeordnet, insbesondere integriert, ist und der zweite Druck- oder Kraftsensor (PBE) an einer Auslauföffnung der ersten Pumpe angeordnet, insbesondere integriert, ist.

**10.** Vorrichtung, die einen extrakorporalen Kreislauf, zumindest einen Druck- oder Kraftsensor (PA, PBE) insbesondere arterieller Druck- oder Kraftsensor und/oder Dialysatoreingangs-Druck- oder Kraftsensor, der in eine Klemmvorrichtung integriert ist, zur Schlauchinnendruckmessung in einem mit Fluid befüllten Schlauch (1) mit arteriellem Abschnitt (1a) und venösem Abschnitt (1b), zumindest einen zum Referenzieren eines Druck- oder Kraftsignals, welches dieser Druck- oder Kraftsensor (PA, PBE) ausgibt, vorgesehenen Kraft- oder Druckreferenzsensor (PHOP, PV) , insbesondere einen arteriellen und/oder venösen Kraft- oder Druckreferenzsensor, aufweist, welcher nicht als Klemmvorrichtung ausgeführt ist und zumindest eine erste Pumpe und eine zweite Pumpe aufweist und welche dazu vorgesehen ist, das Verfahren zur Kalibrierung des Druck- oder Kraftsignals des zumindest einen Druck- oder Kraftsensors (PA, PBE) mithilfe eines Referenzsignals des zumindest einen Kraft- oder Druckreferenzsensors (PHOP, PV) nach einem der vorhergehenden Ansprüche anzuwenden.

**Claims**

**1.** A method for calibrating a first pressure sensor or force sensor (PA) which measures a first pressure of a liquid present inside a tube (1), being a dialyzer tube in an extracorporeal circuit, in the form of a force signal, wherein the first pressure or force sensor (PA) abuts directly on the tube (1) and is integrated into or inserted into a first clamping device, **characterized by** the following steps:

a) regression analysis and prediction of at least one tube-parameter-independent correction function for finding a correction signal for correcting the drift signal caused by clamping of the tube into the first clamping device using a corresponding force or pressure reference signal measured by a first force or pressure reference sensor (PHOP) at a constant internal tube pressure and a constant internal tube temperature;
b) first calibrating of the pressure or force signal, measured by the first pressure or force sensor (PA) and corrected using the correction signal, with the force or pressure reference signal measured by the first force or pressure reference sensor (PHOP) before active use of the tube where the tube is connected to a patient; and
c) second calibrating of the pressure or force signal, measured by the first pressure or force sensor (PA) and corrected using the correction function, with the force or pressure reference signal measured by a second force or pressure reference sensor (PV) during active use of the tube.

**2.** The method for calibration according to claim 1, **characterized in that** in addition to the first pressure, a second pressure, in particular a dialyzer input pressure, measured with a second force sensor or pressure sensor (PBE) integrated in a second clamping device, is measured and corrected; and
the pressure or force signal of the second pressure or force sensor (PBE) is calibrated in the first calibration and in the second calibration with the force or pressure reference signal measured with the second force or pressure reference sensor (PV).

**3.** The method for calibration according to claim 1, **characterized in that** the tube (1) comprises an arterial portion (1a) and a venous portion (1b) and the first and/or second pressure sensor or force sensor (PA, PBE) and, in the case before an active use of the tube (1), the first force or pressure reference sensor (PHOP), are arranged at the arterial portion (1a), while the second force or pressure reference sensor (PV), which does not interact with a clamping device, is arranged at the venous portion (1b).

**4.** The method for calibration according to claim 1, **characterized in that** the first and the second force or pressure reference sensor (PHOP, PV) each have a higher measurement accuracy than the corresponding pressure sensor (PA, PBE).

**5.** The method for calibration according to claim 2, **characterized in that** for calibration of the second force or pressure sensor (PBE) and for calibration of the first and the second force or pressure sensor (PA, PBE) the internal tube pressure is matched, via a bypass circuit in the arterial portion (1a) of the tube (1), with the internal tube pressure in the venous portion (1b) of the tube (1).

**6.** The method for calibration according to claim 1, **characterized in that** the constant internal tube pressure is obtained before active use of the tube by adjusting a pumping ratio between a first pump, in particular a blood pump (BP), and a second pump, in particular a dialysate input flow pump (FPE) and/or a dialysate output flow pump (FPA).

**7.** The method for calibration according to claim 1, **characterized in that** the drift signal is or corresponds to the reset force of the clamped tube (1).

**8.** The method for calibration according to claim 1, **characterized in that** the first and/or second pressure or force signal is converted into a pressure signal by means of the corresponding force or pressure reference signal via a linear recursion.

**9.** The method for calibration according to claim 4, **characterized in that** in the case of two pressure or force sensors used, the first pressure or force sensor (PA) is arranged, in particular integrated, at an inlet opening of the first pump and the second pressure or force sensor (PBE) is arranged, in particular integrated, at an outlet opening of the first pump.

**10.** A device comprising an extracorporeal circuit, at least one pressure or force sensor (PA, PBE), in particular an arterial pressure or force sensor and/or a dialysate inlet pressure or force sensor, integrated in a clamping device, for internal tube pressure measurement in a fluid-filled tube (1) with arterial portion (1a) and venous portion (1b), at least one force or pressure reference sensor (PHOP, PV), in particular an arterial and/or venous force or pressure reference sensor, provided for referencing a pressure or force signal output by this pressure sensor or force sensor (PA, PBE) and which is not designed as a clamping device and comprising at least a first pump and a second pump and which is provided to apply the method for calibrating the pressure or force signal of the at least one pressure or force sensor (PA, PBE) by means of a reference signal of the at least one force or pressure reference sensor (PHOP, PV) according to one of the preceding claims.

## Revendications

**1.** Procédé d'étalonnage d'un premier capteur de pression ou de force (PA) qui mesure, sous la forme d'un signal de force, une première pression d'un liquide qui se trouve à l'intérieur d'un tuyau (1) qui est un tuyau de dialyseur dans un circuit extracorporel, dans lequel le premier capteur de pression ou de force (PA) repose directement contre le tuyau (1) et est intégré dans un premier dispositif de serrage ou est inséré dans celui-ci, **caractérisé par** les étapes suivantes :

a) l'analyse de régression et la prédiction d'au moins une fonction de correction ne dépendant pas de paramètres du tuyau à la recherche d'un signal de correction pour la correction d'un signal de dérive suscité par le serrage du tuyau dans le premier dispositif de serrage à l'aide d'un signal de référence de force ou de pression correspondant qui est mesuré par un premier capteur de référence de force ou de pression (PHOP) en cas de pression interne de tuyau constante et de température interne de tuyau constante ;
b) un premier étalonnage du signal de pression ou de force mesuré avec le premier capteur de pression ou de force (PA) et corrigé à l'aide du signal de correction avec le signal de référence de force ou de pression qui est mesuré par le premier capteur de référence de force ou de pression (PHOP), avant une utilisation active du tuyau, lors de laquelle le tuyau est raccordé à un patient ; et
c) un second étalonnage du signal de pression ou de force mesuré avec le premier capteur de pression ou de force (PA) et corrigé à l'aide de la fonction de correction avec le signal de référence de force ou de pression qui est mesuré par un second capteur de référence de force ou de pression (PV), pendant une utilisation active du tuyau.

2. Procédé d'étalonnage selon la revendication 1, **caractérisé en ce, que** outre la première pression, une seconde pression, en particulier pression d'entrée de dialyseur qui est mesurée avec un second capteur de force ou de pression (PBE) qui est intégré dans un second dispositif de serrage, est mesurée et corrigée ; et le signal de pression ou de force du second capteur de pression ou de force (PBE) est étalonné lors du premier étalonnage et lors du second étalonnage avec le signal de référence de force ou de pression qui est mesuré avec le second capteur de référence de force ou de pression (PV).

3. Procédé d'étalonnage selon la revendication 1, **caractérisé en ce que** le tuyau (1) présente une section artérielle (1a) et une section veineuse (1b) et le premier et/ou second capteur de pression ou de force (PA, PBE) et, dans le cas avant une utilisation active du tuyau (1), le premier capteur de référence de force ou de pression (PHOP), sont ou sont agencés au niveau de la section artérielle (1a) alors que le second capteur de référence de force ou de pression (PV), qui n'interagit pas avec un dispositif de serrage, est ou est agencé au niveau de la section veineuse (1b).

4. Procédé d'étalonnage selon la revendication 1, **caractérisé en ce que** le premier et le second capteur de référence de force ou de pression (PHOP, PV) présente respectivement une précision de mesure supérieure à celle du capteur de pression correspondant (PA, PBE).

5. Procédé d'étalonnage selon la revendication 2, **caractérisé en ce que,** pour l'étalonnage du second capteur de force ou de pression (PBE) et pour l'étalonnage du premier et du second capteur de force ou de pression (PA, PBE), la pression interne de tuyau est ajustée par un circuit de dérivation dans la section artérielle (1a) du tuyau (1) par rapport à la pression interne de tuyau dans la section veineuse (1b) du tuyau (1).

6. Procédé d'étalonnage selon la revendication 1, **caractérisé en ce que** la pression interne de tuyau constante est obtenue avant une utilisation active du tuyau par le réglage d'un rapport de pompe entre une première pompe, en particulier pompe à sang (BP), et une seconde pompe, en particulier pompe à flux d'entrée de dialysat (FPE) et/ou pompe à flux de sortie de dialysat (FPA).

7. Procédé d'étalonnage selon la revendication 1, **caractérisé en ce que** le signal de dérive est la force de rappel du tuyau (1) serré ou correspond à celle-ci.

8. Procédé d'étalonnage selon la revendication 1, **caractérisé en ce que** le premier et/ou second signal de pression ou de force est converti en un signal de pression à l'aide du signal de référence de force ou de pression correspondant par le biais d'une récursion linéaire.

9. Procédé d'étalonnage selon la revendication 4, **caractérisé en ce que,** dans le cas de deux capteurs de pression ou de force utilisés, le premier capteur de pression ou de force (PA) est agencé, en particulier intégré, au niveau d'une ouverture d'entrée de la première pompe et le second capteur de pression ou de force (PBE) est agencé, en particulier intégré, au niveau d'une ouverture de sortie de la première pompe.

10. Dispositif qui présente un circuit extracorporel, au moins un capteur de pression ou de force (PA, PBE) en particulier capteur de pression ou de force artérielle et/ou capteur de pression ou de force d'entrée de dialyseur qui est intégré dans un dispositif de serrage, pour la mesure de pression interne de tuyau dans un tuyau (1) rempli de fluide avec une section artérielle (1a) et une section veineuse (1b), au moins un capteur de référence de force ou de pression (PHOV, PV) prévu pour le référencement d'un signal de pression ou de force qu'émet ce capteur de pression ou de force (PA, PBE), en particulier un capteur de référence de force ou de pression artérielle et/ou veineuse qui n'est pas formé comme dispositif de serrage et présente au moins une première pompe et une seconde pompe et qui est prévu pour appliquer le procédé d'étalonnage du signal de pression ou de force d'au moins un capteur de pression ou de force (PA, PBE) à l'aide d'un signal de référence de l'au moins un capteur de référence de force ou de pression (PHOP, PV) selon l'une quelconque des revendications précédentes.

Fig. 1

Fig. 2A

Fig. 2B

Fig. 3A

Fig. 3B

Fig. 4

Fig. 5

Fig. 6

Fig. 7

Fig. 8

Fig. 9

Fig. 10A

Fig. 10B

Fig. 11A

Fig. 11B

Fig. 12A

Fig. 12B

Fig. 13A

Fig. 13B

**IN DER BESCHREIBUNG AUFGEFÜHRTE DOKUMENTE**

**In der Beschreibung aufgeführte Patentdokumente**

- EP 1357372 A1
- DE 19747254 C2